(19) Europäisches Patentamt
European Patent Office
Office européen des brevets

(11) Publication number: **0 222 022 B1**

(12) **EUROPEAN PATENT SPECIFICATION**

(45) Date of publication of patent specification: **23.09.92**

(51) Int. Cl.5: **C07D 463/00**, C07D 498/053, C07D 499/08, C07D 501/02

(21) Application number: **86903558.4**

(22) Date of filing: **17.05.86**

(86) International application number: **PCT/JP86/00255**

(87) International publication number: **WO 86/06723 (20.11.86 86/25)**

The file contains technical information submitted after the application was filed and not included in this specification

# (54) PROCESS FOR PRODUCING BETA-LACTAM DERIVATIVES.

(30) Priority: **17.05.85 JP 106274/85**

(43) Date of publication of application: **20.05.87 Bulletin 87/21**

(45) Publication of the grant of the patent: **23.09.92 Bulletin 92/39**

(84) Designated Contracting States: **CH DE FR GB IT LI NL**

(56) References cited:
**JP-A-59 104 393**
**JP-B- 4 628 557**
**JP-B- 4 629 864**

(73) Proprietor: **Otsuka Kagaku Kabushiki Kaisha**
**10, Bungo-machi Higashi-ku**
**Osaka-shi Osaka-fu(JP)**

(72) Inventor: **SASAOKA, Michio**
**23-4, Aza Nakasu, Nakamura Kitajimacho, Itano-gun, Tokushima 771-02(JP)**
Inventor: **SAITO, Norio**
**40-38, Aza Nakasao, Shinkirai Kitajimacho, Itano-gun, Tokushima 771-02(JP)**
Inventor: **SHIROI, Takashi**
**72-2, Aza Motosu, Nakamura Kitajimacho, Itano-gun, Tokushima 771-02(JP)**
Inventor: **NAGAO, Shigemitsu**
**6-76, Showa-cho, Tokushima-shi, Tokushima 770(JP)**
Inventor: **KIKUCHI, Ryo**
**10-56, Sumiyoshi 4-chome, Tokushima-shi, Tokushima 770(JP)**
Inventor: **KAMEYAMA, Yutaka**
**463-10, Kagasuno, Kawauchi-cho, Tokushima-shi, Tokushima 770(JP)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 222 022 B1

(74) Representative: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**W-8000 München 40(DE)**

## Description

### Technical Field

The present invention relates to a method for production of β-lactam derivatives.

### Background Art

β-Lactam derivatives used as antibiotics generally have carboxyl groups within their molecules and, in many instances, are put to use as the free carboxylic acids or with the carboxyl groups protected in the form of pharmaceutically acceptable salts. However, in the process of synthesis of β-lactam antibiotics, such carboxyl groups are usually protected with suitable protective groups, which must be ultimately eliminated in good yield without affecting the other moiety of the molecule.

Heretofore, elimination of the carboxyl-protecting group X from a β-lactam derivative having a protected carboxyl group, which may be represented by the general formula:

A-COO-X (II)

wherein A means a β-lactam derivative residue, and X means a benzyl group having an electron-donating group as a phenyl ring substituent, a diphenylmethyl group which may have an electron-donating group as a phenyl ring substituent, or a tert-butyl group, to give a β-lactam derivative of the general formula:

A-COOH (I)

wherein A is as defined above is accomplished by subjecting the β-lactam derivative of general formula (II) to catalytic reduction using a noble metal catalyst or to treatment with an acid, for instance. The latter method includes such versions as one using trifluoroacetic acid (Journal of The American Chemical Society 91, 5674, 1969), one using formic acid (Chemical Pharmaceutical Bulletin 30, 4545, 1982), the method in which the starting compound is reacted with aluminum chloride in the presence of anisole (Tetrahedron Letters 2793, 1979) and so on. However, these prior art methods have the following disadvantages.

Referring, first, to the catalytic reduction using a noble metal catalyst, β-lactam antibiotics generally contain a sulfide bond which may act as a catalyst poison and, therefore, the expensive noble metal catalyst must be used in a large amount. Furthermore, this method cannot be applied to β-lactam antibiotics containing reducible groups such as nitro, a carbon-to-carbon multiple bond or the like. Moreover, when the protective group is a tert-butyl group, it cannot be eliminated by this method. Further, when the protective group is a benzyl group having an electron-donating group as a phenyl ring substituent or a diphenylmethyl group having an electron-donating group as a phenyl ring substituent, this method fails to eliminate these groups in many instances.

The method using an acid also has the disadvantage that it requires at least a stoichiometric amount of strong acid despite the fact that the β-lactam derivative of general formula (I) is unstable to acid, with the result that the β-lactam derivative of general formula (I) once produced by the method is decomposed to detract from its ultimate yield.

For example, when a p-methoxybenzyl group masking the carboxyl group of a cephalosporin compound of the formula (III) given below is to be eliminated with trifluoroacetic acid, this expensive reagent trifluoroacetic acid must be used generally in an amount of at least 5 molar equivalents relative to the cephalosporin compound of general formula (III). With an equimolar amount of trifluoroacetic acid, for instance, the above reaction does not substantially proceed. Suppose that the cephalosporin of formula (III) were deprotected with a large amount of trifluoroacetic acid and, after the reaction, one tried to recover the trifluoroacetic acid for reuse, a substantial loss of the acid would be inevitable. Furthermore, in the course of the recovery procedure, the acid-labile compound of the following formula (IV) would be decomposed to further detract from the reaction yield.

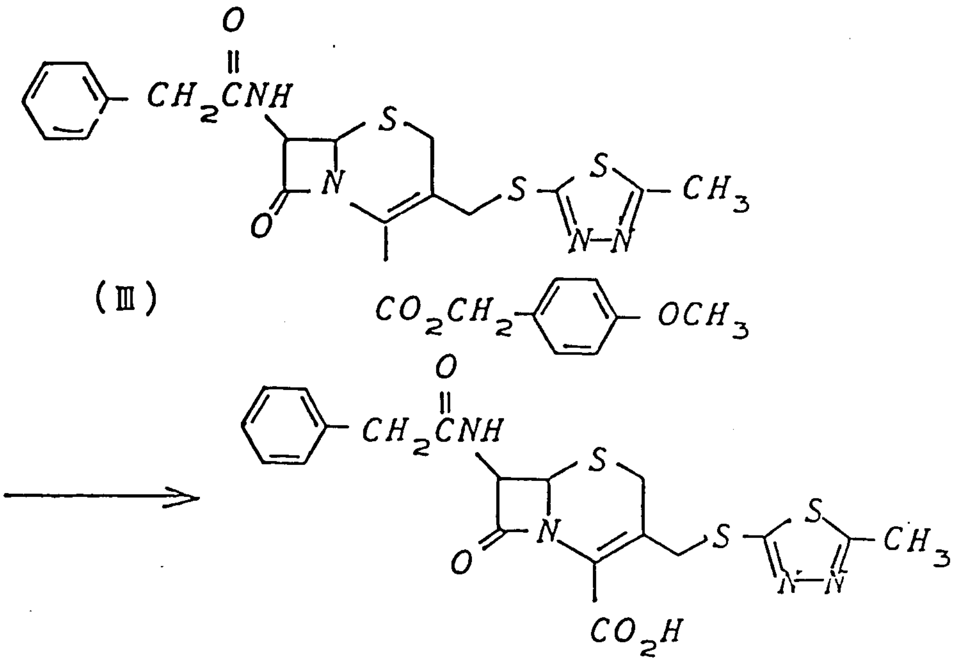

(III)

(IV)

The method employing formic acid also has a similar disadvantage. Thus, the expensive reagent formic acid of 98 to 100% concentration must be used in large excess as a reaction solvent. And if vacuum distillation, for instance, be carried out for its recovery and reuse, the acid-labile compound of the above formula (IV) would be decomposed to detract from its yield.

The method which comprises reacting the protected compound with aluminum chloride in the presence of anisole has the following disadvantages. It is essential to use aluminum chloride which, however, is difficult to handle because it tends to undergo exothermic reaction with atmospheric moisture to give rise to hydrochloric acid. Furthermore, as the reaction mixture is rendered strongly acidic during the reaction or the subsequent workup procedure, the acid-labile compound of general formula (IV) is decomposed so that the yield of the compound is adversely affected. In addition, in the workup procedure after the reaction, a large amount of aluminum hydroxide must be disposed of.

It is, thus, clear that there is not available a method by which a carboxyl-protecting group X can be eliminated from a carboxy-protected $\beta$-lactam derivative of general formula (II) to give a $\beta$-lactam derivative of general formula (I) in good yield and without difficulties on a commercial scale.

## Disclosure of Invention

The present invention provides a method by which a carboxyl-protecting group X can be eliminated from a carboxy-protected $\beta$-lactam derivative of general formula (II) to give a $\beta$-lactam derivative of general formula (I) in good yield and without difficulties on a commericial scale.

Thus, the present invention relates to a method of producing a $\beta$-lactam derivative characterized by reacting a carboxy-protected $\beta$-lactam derivative of general formula (II) with a phenol compound to give a $\beta$-lactam derivative of general formula (I). As examples of the $\beta$-lactam derivative residue designated by A in this specification, there may be mentioned the groups represented by the following general formula.

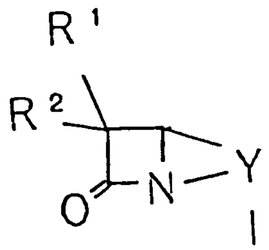

(V)

wherein 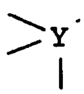 means 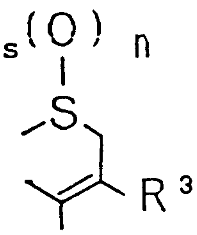 , 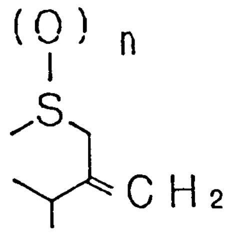 , 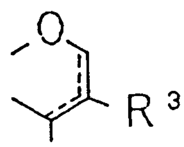 ,

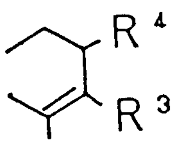 or 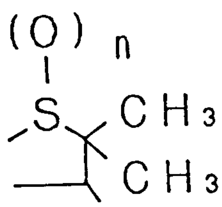

More particularly, the following groups may be mentioned by way of example.

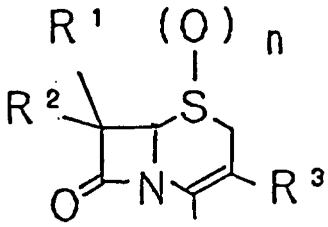

...... Cephalosporin derivative residues

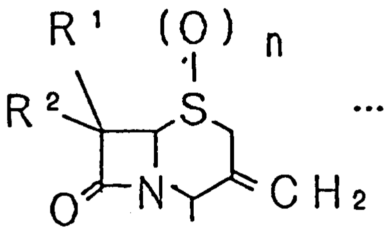

...... 3-Exomethylenecepham derivative residues

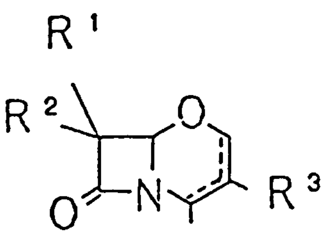

...... 1-Oxacephem derivative residues

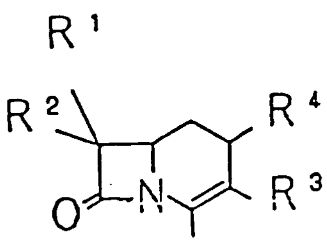

...... 1-Carbacephem derivative residues

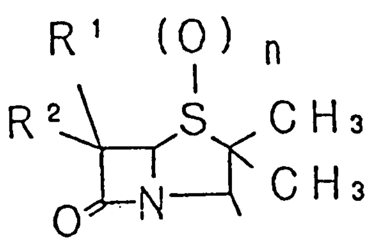

... Penicillin derivative residues

In the above residues, $R^1$ may for example be any of the substituents in 7-position of the known cephalosporins. To be specific, a hydrogen atom, a lower alkoxy group such as methoxy, ethoxy, etc., and a formamido group may be mentioned as examples.

Examples of $R^2$ include the substituents in 6-position of the known penicillins or in 7-position of the known cephalosporins such as mentioned in Mary C. Griffiths (ed.): USAN and the USP Dictionary of Drug Names. To be specific, a hydrogen atom, a halogen atom, an amino group, an amido group and so on may be mentioned. The halogen atom mentioned just above may for example be fluorine, chlorine, bromine or iodine. As examples of said amido group, there may be mentioned benzamido, phenylacetamido, phenoxyacetamido, phenylglycylamido, amino-protected phenylglycylamido, p-hydroxyphenylglycylamido, hydroxy- and amino-protected p-hydroxyphenylglycylamido, thiolacetamido, α-carboxyp henylacetamido,carboxy-protected α-carboxyphenylacetamido, α-hydroxyphenylacetamido, hydroxy-protected α-hydroxyphenylacetamido, 2-(2-amino-4-thiazolyl)-2-methoxyiminoacetamido, amino-protected 2-(2-amino-4-thiazolyl)-2-methoxyiminoacetamido, 2-(2-furyl)-2-methoxyiminoacetamido, 2-(2-amino-4-thiazolyl)-2-(carboxymethoxyimino)acetamido, amino- and carboxy-protected 2-(2-amino-4-thiazolyl)-2-(carboxymethoxyimino)acetamido, α-alanyloxyphenylacetamido, tetrazolylacetamido, α-(4-ethyl-2,3-dioxo-1-piperazinocarbonylamido-p-hydroxyphenylacetamido, hydroxy-protected α-(4-ethyl-2,3-dioxo-1-piperazinocarbonylamido)-p-hydroxyphenylacetamido, o-aminomethylphenylacetamido, amino-protected o-aminomethylphenylacetamido, 2-(5-carboxyimidazole-4-carboxyamido)-2-phenylacetamido, carboxy-protected 2-(5-carboxyimidazole-4-carboxyamido)-2-phenylacetamido, 2-(4-hydroxy-6-methylnicotinamido)-2-(p-hydroxyphenyl)acetamido, hydroxy-protected 2-(4-hydroxy-6-methylnicotinamido)-2-(p-hydroxyphenyl)-acetamido, α-sulfophenylacetamido, 2-(2-amino-4-thiazolyl)-2-(1-carboxy-1-methylethyloxyimino)acetamido, amino- and carboxy-protected 2-(2-amino-4-thiazolyl)-2-(1-carboxy-1-methyloxyimino)acetamido, 2-(6,7-dihydroxy-4-oxo-1-benzopyran-3-carboxyamido)-2-(p-hydroxyphenyl)acetamido, hydroxy-protected 2-(6,7-dihydroxy-4-oxo-1-benzopyran-3-carboxyamido)-2-(p-hydroxyphenyl)acetamido, cyanomethylthioacetamido, 2-aminocarbonyl-2-fluorovinyltioacetamido, amino-protected 2-aminocarbonyl-2-fluorovinylthioacetamido, difluoromethylthioacetamido, α-carboxy-α-(p-hydroxyphenyl)acetamido, hydroxy- and carboxy-protected α-carboxy-α-(p-hydroxyphenyl)acetamido, and so on. As the protective groups for said amino, hydroxyl and carboxyl groups, many known protective groups may used. To be specific, there may be mentioned benzyl, p-methoxybenzyl, p-nitrobenzyl, 3,4,5-trimethoxybenzyl, 3,5-dimethoxy-4-hydroxybenzyl, 2,4,6-trimethylbenzyl, diphenylmethyl, ditolylmethyl, trityl; piperonyl, naphthylmethyl, 9-anthrylmethyl, tert-butyl, trichloroethyl, and so on.

As examples of $R^3$, the known substituents in 3-position of cephalosporins as mentioned in the USAN and the USP Dictionary of Drug Names may be mentioned. To be specific, hydrogen atom, hydroxyl group, halogen atoms such as chlorine, bromine, fluorine, etc., lower alkoxyl groups such as methoxy, ethoxy, etc., substituted and unsubstituted vinyl groups such as vinyl, 2,2-dibromovinyl, etc., ethynyl group, lower alkyl groups such as methyl, ethyl, etc., lower alkoxymethyl groups such as methoxymethyl, ethoxymethyl, etc., acetoxymethyl group, carbamoyloxymethyl group, heterocycle-thiomethyl groups such as 1,2,3,-triazol-4-ylthiomethyl, 5-methyl-1,3,4-thiadiazol-2-ylthiomethyl, 1-methyltetrazol-5-ylthiomethyl, 1-sulfomethyltetrazol-5-ylthiomethyl, 1-carboxymethyltetrazol-5-ylthiomethyl, 1-(2-dimethylaminoethyl)tetrazol-5-ylthiomethyl, 1,2,3-thiadiazol-5-ylthiomethyl, 1-(2-hydroxyethyl)tetrazol-5-ylthiomethyl, etc., 5-methyltetrazol-2-ylmethyl group, 1-methylpyrrolidinomethyl group, pyridiniummethyl group and so on may be mentioned.

As examples of $R^4$, there may be mentioned the substituents in 2-position of the known 1-carbacephem compounds. To be specific, hydrogen atom, hydroxyl group, halogen atoms such as chlorine, bromine, etc., lower alkoxyl groups such as methoxy, ethoxy, etc., lower acyloxyl groups such as formyloxy, acetyloxy, propionyloxy, etc., lower alkylthio groups such as methylthio, ethylthio, etc., heterocycle-thio groups such as 1,3,4-thiadiazol-2-ylthio, 5-methyl-1,3,4-thiadiazol-2-ylthio, 1-methyltetrazol-5-ylthio, etc. and so on may be mentioned.

The symbol n means 0, 1 or 2.

In this specification, the electron-donating group as a substituent on the phenyl ring of the benzyl or diphenylmethyl group represented by X is exemplified by hydroxyl group, lower alkyl groups such methyl, ethyl, tert-butyl, etc., and lower alkoxyl groups such as methoxy, ethyoxy, and so on. In regard to said diphenylmethyl group, groups such that substituted or unsubstituted phenyl groups are bound together through a methylene chain or a hetero-atom within the molecule are included. As examples of the protective group in the present invention, there may be mentioned p-methoxybenzyl, diphenylmethyl, 3,4,5-trimethoxybenzyl, 3,5-dimethoxy-4-hydroxybenzyl, 2,4,6-trimethylbenzyl, piperonyl, ditolylmethyl, naphthylmethyl, 9-anthrylmethyl, tert-butyl and other groups.

As the β-lactam derivative of general formula (II) used in accordance with the present invention, any of the known derivatives subsumed in the above general formula can be employed.

The phenol compound used in accordance with the present invention is substituted or unsubstituted phenol, and substituents on the phenyl ring may for example be halogen atoms such as chlorine, bromine, etc., lower alkyl groups such as methyl, ethyl, etc., and lower alkoxyl groups such as methoxy, ethoxy, and so on. Examples of such substituted phenols include o-chlorophenol, m-chlorophenol, p-chlorophenol, o-cresol, m-cresol, p-cresol, m-methoxyphenol, and so on.

In the present invention, a β-lactam derivative of general formula (II) is reacted with said phenol compound. Since phenol compound can be used not only as the reactant but also as the solvent, there is no particular limitation on its amount but a suitable amount can be chosen from a broad range. Generally,

about 0.5 to 500 weight parts, preferably about 1 to 200 weight parts, can be used relative to each weight part of the β-lactam derivative of general formula (II).

Generally the reaction according to the present invention is preferably conducted in the molten state of said phenol compound and although a solvent is not essential, the reaction may be carried out in the presence of a co-solvent which may be water or an organic solvent such as a halogenated hydrocarbon, e.g. carbon tetrachloride, chloroform, methylene chloride, dichloroethane, trichloroethane, dichloroethylene and so on. However, the use of a large amount of such solvent may interfere with the above reaction and is, therefore, undesirable. The amount of water or said organic solvent is about the same as the amount of phenol compound at most.

The reaction temperature for the above reaction depends on the type of β-lactam derivative of general formula (II), the type of phenol compound, and other factors, and cannot be stated in general terms. However, the reaction temperature is preferably in the range of the minimum temperature at which no solidification of the reaction system takes place to about 100 °C and, for still better results, in the range of the minimum temperature at which no solidification takes place to about 70 °C.

In the present invention, a catalyst amount of acid may be present within the reaction system. In this case, depending on types of β-lactam derivatives, the above reaction may be carried through at a lower temperature and in a shorter time. The catalyst amount mentioned above varies with the type of β-lactam derivative and the type of acid but generally speaking, about 0.01 to 100 mole percent, preferably about 0.01 to 50 mole percent, of acid is used relative to the β-lactam derivative of general formula (II). As examples of the acid used as the catalyst, there may be mentioned mineral acids such as hydrochloric acid, sulfuric acid, perchloric acid, phosphoric acid, etc., carboxylic acids such as formic acid, acetic acid, monochloroacetic acid, dichloroacetic acid, trichloroacetic acid, monofluoroacetic acid, difluoroacetic acid, trifluoroacetic acid, etc., sulfonic acids such as benzenesulfonic acid, p-toluenesulfonic acid, metanesulfonic acid, etc., acidic enol compounds such as meldrum's acid, squaric acid, etc., Lewis acids such as aluminum chloride, and solid acids such as acid sodium sulfate, acid potassium sulfate, acid ion exchange resins, and so on.

Following completion of the above reaction, the reaction mixture may be after-treated in the conventional manner to isolate the product β-lactam derivative of general formula (I). For example, either sodium hydrogen carbonate or sodium carbonate and a hydrophobic organic solvent are added to the reaction mixture to extract the β-lactam derivative of general formula (I) into the aqueous layer or crystallized with a bad organic solvent followed by the conventional after-treatment to give the β-lactam derivative of general formula (I) in high yield. Further, by distilling the organic layer, the extraction solvent and the phenol compound can be recovered for re-use.

In accordance with the method of the present invention, the protective group can be easily eliminated from the carboxy-protected β-lactam derivative (II). Moreover, since the present invention does not required a large amount of acid unlike in the conventional method, the β-lactam derivative (I) produced by the above reaction is little decomposed, with the result that the β-lactam derivative (I) can be produced in high yield. In addition, because the phenol compound, extraction solvent, etc. can be efficiently recovered, the present invention is economically advantageous. Incidentally, when the moiety A of the β-lactam derivative (II) treated according to the present invention has a protected carboxyl group, it may also be eliminated at the same time.

## Examples

The following comparative and working examples are intended to illustrate the present invention in further detail.

### Comparative Example 1

Ten grams of a compound of general formula (II) (where A = cephalosporin derivative residue; X = p-methoxybenzyl; $R^1$ = H; $R^2$ = phenylacetamido; $R^3$ = 5-methyl-1,3,4-thiadiazol-2-ylthiomethyl; n = 0), 100 ml of methylene chloride and 6.61 ml of trifluoroacetic acid were admixed and reacted at room temperature for 2 hours. Following completion of the reaction, the methylene chloride and trifluoroacetic acid were distilled off under reduced pressure. To the concentration residue were added 100 ml of 5% aqueous sodium carbonate and 150 ml of ethyl acetate for extraction. The water layer was adjusted to pH 1-2 with hydrochloric acid under ice-cooling and extracted with 300 ml of ethyl acetate. The resulting ethyl acetate layer was concentrated under reduced pressure to give the compound of general formula (I) (wherein A = cephalosporin derivative residue; $R^1$ = H; $R^2$ = phenylacetamido; $R^3$ = 5-methyl-1,3,4-thiadiazol-2-ylthiomethyl; n=0) in a yield of 61%. The NMR spectrum of this product was in agreement with that of the compound obtained in Example 1 which appear hereinafter. The trifluoroacetic acid distilled off under reduced pressure was trapped with liquid nitrogen and determined by titrimetry. The recovered amount was 3.44 ml.

Comparative Example 2

Ten grams of a compound of general formula (II) (where A = cephalosporin derivative residue; X = p-methoxybenzyl; $R^1$ = H; $R^2$ = phenylacetamido; $R^3$ = 5-methyl-1,3,4-thiadiazol-2-ylthiomethyl; n=0), 100 ml of methylene chloride, and 1,32 ml of trifluoroacetic acid were admixed and reacted at room temperature for 5 hours. The reaction did not proceed appreciably.

Comparative Example 3

Ten grams of a compound of general formula (II) (where A = cephalosporin derivative residue; X = p-methoxybenzyl; $R^1$ = H; $R^2$ = phenylacetamido; $R^3$ = 5-methyl-1,3,4-thiadiazol-2-ylthiomethyl; n=0) and 100 ml of 99% formic acid were admixed and reacted at room temperature for 3 hours. Following completion of the reaction, the formic acid was distilled off. The concentration residue was after-treated in the same manner as Comparative Example 1 to give a compound of general formula (I) (where A = cephalosporin derivative residue; $R^1$ = H; $R^2$ = phenylacetamido; $R^3$ = 5-methyl-1,3,4-thiadiazol-2-ylthiomethyl; n=0) in a yield of 55%. The NMR spectrum of this compound was in good agreement with that of the compound produced in Example 1 which appears hereinafter.

Comparative Example 4

In the same manner as described in Tetrahedron Letters 2793 (1979), 1 gram of a compound of general formula (II) (where A = cephalosporin derivative residue; X = p-methoxybenzyl; $R^1$ = H; $R^2$ = phenylacetamido; $R^3$ = 5-methyl-1,3,4-thiadiazol-2-ylthiomethyl; n=0) was reacted with aluminum chloride to give a compound of general formula (I) (where A = cephalosporin derivative residue; $R^1$ = H; $R^2$ = phenylacetamido; $R^3$ = 5-methyl-1,3,4-thiadiazol-2-ylthiomethyl; n=0) in a yield of 62%. The NMR spectrum of this compound was in agreement with the compound obtained in Example 1 which appears below.

Example 1

Two grams of a compound of general formula (II) (A = cephalosporin derivative residue; X = p-methoxybenzyl; $R^1$ = hydrogen; $R^2$ = phenylacetamido; $R^3$ = 5-methyl-1,3,4-thiadiazol-2-ylthiomethyl; n=0) and 6.5 ml of phenol were heated at 60°C for 3 hours. After the reaction, the reaction mixture was allowed to cool to room temperature and extracted with 19.5 ml of ethyl acetate and 15 ml of 5% aqueous sodium carbonate solution. The water layer was adjusted to pH 1-2 with hydrochloric acid under cooling in an ice bath and extracted with 30 ml of ethyl acetate. The ethyl acetate layer was then concentrated under reduced pressure to give a compound of general formula (I) (A = cephalosproin derivative residue; $R^1$ = hydrogen; $R^2$ = phenylacetamido; $R^3$ = 5-methyl-1,3,4-thiadiazol-2-ylthiomethyl; n=0) in a yield of 85%. The NMR spectrum of the product compound supported its structure.

NRM (acetone-$d_6$), $\delta$ ppm:
2.70 (s, 3H)
3.65 (s, 2H)
3.75 (bs, 2H)
4.35 and 4.53 (ABq, 2H, J = 14 Hz)
5.08 (d, 1H, J = 5 Hz)
5.78 (dd, 1H, J = 5 Hz, 9 Hz)
7.27 (bs, 5H)
7.87 (d, 1H, J = 5 Hz)

Example 2

Twenty grams of a compound of general formula (II) (A = cephalosporin derivative residue; X = p-methoxybenzyl; $R^1$ = hydrogen; $R^2$ = phenylacetamido; $R^3$ = 5-methyl-1,3,4-thiadiazol-2-ylthiomethyl;

n = 0) and 40 ml of phenol were mixed and heated at 40 ° C, followed by addition of 0.25 ml of concentrated hydrochloric acid. The reaction was conducted for 1 hour. After completion of the reaction, the reaction mixture was allowed to cool to room temperature and extracted with 160 ml of methyl isobutyl ketone and 75 ml of 5% aqueous sodium carbonate solution. Under cooling in an ice bath, the aqueous layer was adjusted to pH 1-2 with hydrochloric acid and extracted with 300 ml of ethyl acetate. The ethyl acetate layer was concentrated under reduced pressure to give a compound of general formula (I) (A = cephalosporin derivative residue; $R^1$ = H; $R^2$ = phenylacetamido; $R^3$ = 5-methyl-1,3,4-thiadiazol-2-ylthiomethyl; n = 0) in a yield of 90%. The NMR spectrum of this product compound was in good agreement with that of the compound obtained in Example 1.

## Example 3

Twenty grams of a compound of general formula (II) (A = cephalosporin derivative residue; X = p-methoxybenzyl; $R^1$ = H; $R^2$ = phenylacetamido; $R^3$ = 1-methyltetrazol-5-ylthiomethyl; n = 0) and 40 ml of phenol were mixed and heated at 45 ° C, followed by addition of 85 μl of concentrated hydrochloric acid. The reaction was conducted for 1 hour. After completion of the reaction, the reaction mixture was worked up in the same manner as Example 2 to give a compound of general formula (I) (A = cephalosporin derivative residue; $R^1$ = H; $R^2$ = phenylacetamido; $R^3$ = 1-methyltetrazol-5-ylthiomethyl; n = 0) in a yield of 92%. The NMR spectrum of this product compound supported its structure.

NMR (acetone-$d_6$) δ ppm:
3.65 (s, 2H)
3.75 (s, 2H)
3.98 (s, 3H)
4.37 (s, 2H)
5.06 (d, 1H J = 5 Hz)
5.74 (dd, 1H, J = 5 Hz, 9 Hz)
7.27 (bs, 5 H)
7.90 (d, 1H, J = 9 Hz)

## Example 4

Twenty grams of a compound of general formula (II) (A = cephalosporin derivative residue; X = diphenylmethyl; $R^1$ = H; $R^2$ = phenylacetamido; $R^3$ = 1-methyltetrazol-5-ylthiomethyl; n = 0) and 40 ml of phenol were mixed and heated at 45 ° C, followed by addition of 80 μl of concentrated hydrochloric acid. The reaction was conducted for 1 hour. After the reaction, the reaction mixture was worked up in the same manner as Example 2 to give a compound of general formula (I) (A = cephalosporin derivative residue; $R^1$ = H; $R^2$ = phenylacetamido; $R^3$ = 1-methyltetrazol-5-ylthiomethyl; n = 0) in a yield of 95%. The NMR spectrum of this product compound was in agreement with that of the compound obtained in Example 3.

## Example 5

Twenty grams of a compound of general formula (II) (A = cephalosporin derivative residue; X = p-methoxybenzyl; $R^1$ = H; $R^2$ = phenylacetamido; $R^3$ = acetoxymethyl; n = 0) and 40 ml of phenol were mixed and heated at 45 ° C. To this mixture was added 100 μl of concentrated hydrochloric acid and the reaction was conducted for 1 hour. After completion of the reaction, the reaction mixture was worked up in the same manner as Example 2 to give a compound of general formula (I) (A = cephalosporin derivative residue; $R^1$ = H; $R^2$ = phenylacetamido; $R^3$ = acetoxymethyl; n = 0) in a yield of 93%. The NMR spectrum of this product compound supported its structure.

NMR (acetone-$d_6$) δ ppm:
2.04 (s, 3H)
3.52 and 3.61 (ABq, 2H, J = 17 Hz)
3.67 (s, 2H)
4.86 and 5.04 (ABq, 2H, J = 14 Hz)
5.08 (d, 1H, J = 5 Hz)
5.35-6.50 (bs, 3H)
5.80 (dd, 1H, J = 5 Hz, 8 Hz)
7.27 (bs, 5 H)
7.87 (d, 1H, J = 8 Hz)

Example 6

The procedure of Example 5 was repeated except that compounds of general formula (II) (A = cephalosporin derivative residue; $R^1$ = H; $R^2$ = phenylacetamido; $R^3$ = acetoxymethyl; n = 0) having the under-mentioned species of X were used. The resulting product compounds of general formula (I) and their yields were as follows.

- The compound of general formula (II) wherein X = 3,4,5-trimethoxybenzyl ..... Yield 90%.
- The compound of general formula (II) wherein X = 3,5-dimetoxy-4-hydroxybenzyl ..... Yield 85%.
- The compound of general formula (II) wherein X = 2,4,6-trimethylbenzyl ..... Yield 88%.
- The compound of general formula (II) wherein X = 3,4-methylenedioxybenzyl ..... Yield 90%.
- The compound of general formula (II) wherein X = diphenylmethyl ..... Yield 94%.
- The compound of general formula (II) wherein X = ditolylmethyl ..... Yield 96%.
- The compound of general formula (II) wherein X = tert-butyl ..... Yield 84%.

Example 7

By the same procedure as Example 3 except that one of the acids mentioned below in Table 1 was used as a catalyst and the reaction conditions specifically indicated in Table 1 were employed, a compound of general formula (II) (A = cephalosporin derivative residue; X = p-methoxybenzyl; $R^1$ = H; $R^2$ = phenylacetamido; $R^3$ = 1-methyltetrazol-5-ylthiomethyl; n = 0) was treated to give the corresponding compound of general formula (I) (A = cephalosporin derivative residue; $R^1$ = H; $R^2$ = phenylacetamido; $R^3$ = 1-methyltetrazol-5-ylthiomethyl; n = 0).

Table 1

| Acid | Amount of acid (based on starting compound) (mole %) | Reaction time (hr) | Reaction temperature (° C) | Yield (%) |
|---|---|---|---|---|
| Sulfuric acid | 2 | 1 | 45 | 96 |
| Perchloric acid | 0.2 | 1 | 45 | 91 |
| Trifluoroacetic acid | 2 | 1.5 | 60 | 89 |
| Trifluoroacetic acid | 10 | 1.5 | 60 | 96 |
| Trichloroacetic acid | 10 | 1.5 | 60 | 90 |
| p-Toluenesulfonic acid | 5 | 1 | 45 | 94 |
| Methanesulfonic acid | 2 | 1 | 45 | 95 |

Example 8

Two-hundred milligrams of a compound of general formula (II) (A = cephalosporin derivative residue; X = p-methoxybenzyl; $R^1$ = H; $R^2$ = phenylacetamido; $R^3$ = 1-methyltetrazol-5-ylthiomethyl; n = 0) and 0.65 ml of m-chlorophenol were mixed and heated at 60° C for 1 hour. After completion of the reaction, the reaction mixture was worked up in the same manner as Example 1 to give a compound of general formula (I) (A = cephalosporin derivative residue; $R^1$ = H; $R^2$ = phenylacetamido; $R^3$ = 1-methyltetrazol-5-ylthiomethyl; n = 0) in a yield of 90%. The NMR spectrum of this product compound was in agreement with that of the compound obtained in Example 3.

Example 9

Two grams of a compound of general formula (II) (A = cephalosporin derivative residue; X = p-methoxybenzyl; $R^1$ = H; $R^2$ = phenylacetamido; $R^3$ = 1-methyltetrazol-5-ylthiomethyl; n = 0) and 6.5 ml of m-chlorophenol, and 17 $\mu$l of concentrated hydrochloric acid were mixed and heated at room temperature for 3 hours. After completion of the reaction, the reaction mixture was worked up in the same manner as Example 2 to give a compound of general formula (I) (A = cephalosporin derivative residue; $R^1$ = H; $R^2$ = phenylacetamido; $R^3$ = 1-methyltetrazol-5-ylthiomethyl; n = 0) in a yield of 89%. The NMR spectrum of this product compound was in agreement with that of the compound obtained in Example 3.

Example 10

A compound of general formula (II) (A = cephalosporin derivative residue; X = diphenylmethyl; $R^1$ = H; $R^2$ = phenylacetamido; $R^3$ = acetoxymethyl; n=0) was treated in the same manner as Example 2 to give a compound of general formula (I) (A = cephalosporin derivative residue; $R^1$ = H; $R^2$ = phenylacetamido; $R^3$ = acetoxymethyl; n=0) in a yield of 93%. The NMR spectrum of this product compound supported its chemical structure.
NMR ($CDCl_3$) δ ppm:
2.10 (s, 3H)
3.43 and 3.53 (ABq, 2H, J = 19 Hz)
4.57 (s, 2H)
4.92 and 5.08 (ABq, 2H, J = 15 Hz)
5.02 (d, 1H, J = 5 Hz)
5.87 (dd, 1H, J = 5 Hz, 9 Hz)
6.75-7.50 (m, 6H)

Example 11

A compound of general formula (II) (A = cephalosporin derivative residue; X = p-methoxybenzyl; $R^1$ = H; $R^2$ = tetrazolylacetamido; $R^3$ = 5-methyl-1,3,4-thiadiazol-2-ylthiomethyl; n=0) was treated in the same manner as Example 2 to give a compound of general formula (I) (A = cephalosporin derivative residue; $R^1$ = H; $R^2$ = tetrazolylacetamido; $R^3$ = 5-methyl-1,3,4-thiadiazol-2-ylthiomethyl; n=0) in a yield of 86%. The NMR spectrum of this product compound supported its chemical structure.
NMR (DMSO-$d_6$) δ ppm:
2.70 (s, 3H)
3.00-5.00 (bs, 1H)
3.63 and 3.76 (ABq, 2H, J = 18 Hz)
4.23 and 4.50 (ABq, 2H, J = 14 Hz)
5.10 (d, 1H, J = 5 Hz)
5.36 (s, 2H)
5.70 (dd, 1H J = 5 Hz, 8 Hz)
9.31 (s, 6H)
9.43 (d, 1H, J = 8 Hz)

Example 12

A compound of general formula (II) (A = cephalosporin derivative residue; X = p-methoxybenzyl; $R^1$ = H; $R^2$ = α-(tert-butyloxycarbonylamino)phenylacetamido; $R^3$ = 5-methyl-1,3,4-thiadiazol-2-ylthiomethyl; n=0) was treated in the same manner as Example 2 to give a compound of general formula (I) (A = cephalosporin derivative residue; $R^1$ = H; $R^2$ = α-(tert-butyloxycarbonylamino)phenylacetamido; $R^3$ = 5-methyl-1,3,4-thiadiazol-2-ylthiomethyl; n=0) in a yield of 89%. The NMR spectrum of this product compound supported its chemical structure.
NMR (DMSO-$d_6$) δ ppm:
1.36 (s, 9H)
3.25-4.50 (bs, 1H)
3.56 (bs, 2H)
3.91 (s, 3H)
4.00 (d, 1H, J = 8 Hz)
4.22 (bs, 2H)
4.94 (d, 1H, J = 5 Hz)
5.26 (d, 1H, J = 8 Hz)
5.65 (dd, 1H, J = 5 Hz, 8 Hz)
7.10-7.55 (m, 5 H)
9.07 (d, 1H, J = 8 Hz)

Example 13

A compound of general formula (II) (A = cephalosporin derivative residue; X = p-methoxybenzyl; $R^1$ =

H; $R^2$ = amino; $R^3$ = 5-methyl-1,3,4-thiadiazol-2-ylthiomethyl; n = 0) was reacted in the same manner as Example 2. Following the reaction, ethyl acetate was gradually added with stirring whereupon a compound of general formula (I) (A = cephalosporin derivative residue; $R^1$ = H; $R^2$ = amino; $R^3$ = 5-methyl-1,3,4-thiadiazol-2-ylthiomethyl; n = 0) separated out. The precipitate was recovered by filtration and purified in the conventional manner. The above procedure gave the object compound in a yield of 84%. The NMR spectrum of this product compound was in agreement with the NMR spectrum described in the literature.

Example 14

Ten grams of a compound of general formula (II) (A = exomethylenecepham derivative residue; X = p-methoxybenzyl; $R^1$ = H; $R^2$ = phenylacetamido; n = 0) and 20 ml of phenol were mixed and heated at 45 ° C. To this mixture was added 0.37 ml of concentrated hydrochloric acid and the reaction was conducted for 1 hour. After completion of the reaction, the reaction mixture was allowed to cool to room temperature and extracted with 150 ml of ethyl acetate and 100 ml of a saturated aqueous solution of sodium carbonate. Under-cooling in an ice bath, the aqueous layer was adjusted to pH 1-2 with hydrochloric acid and extracted with 200 ml of ethyl acetate. The ethyl acetate layer was concentrated under reduced pressure to give a compound of general formula (I) (A = 3-exomethylenecephem derivative residue; $R^1$ = H; $R^2$ = phenylacetamido; n = 0) in a yield of 85%. The NMR spectrum of this product compound supported its chemical structure.

NMR (acetone-$d_6$) $\delta$ ppm:
3.35 and 3.69 (ABq, 2H, J = 14 Hz)
3.65 (s, 2H)
5.08 (s, 1H)
5.28 (s, 2H)
5.34 (d, 1H, J = 5 Hz)
5.57 (dd, 1H, J = 5 Hz, 9 Hz)
7.05-7.45 (m, 5H)
7.75 (d, 1H, J = 9 Hz)

Example 15

A compound of general formula (II) (A = 3-exomethylenecepham derivative residue; X = p-methoxybenzyl; $R^1$ = H; $R^2$ = phenoxyacetamido; n = 1) was treated in the same manner as Example 14 to give a compound of general formula (I) (A = 3-exomethylenecepham derivative residue; $R^1$ = H; $R^2$ = phenoxyacetamido; n = 1) in a yield of 87%. The NMR spectrum of this product compound supported its chemical structure.

NMR (DMSO-$d_6$/$CDCl_3$) $\delta$ ppm:
3.80 (s, 2H)
4.53 (s, 2H)
5.05 (s, 1H)
5.10 (d, 1H, J = 5 Hz)
5.40 (s, 1H)
5.70 (s, 1H)
5.90 (dd, 1H, J = 5 Hz, 9 Hz)
6.85-7.50 (m, 5H)
9.65 (d, 1H, J = 9 Hz)

Example 16

A compound of general formula (II) (A = cephalosporin derivative residue; X = diphenylmethyl; $R^1$ = H; $R^2$ = thiolacetamido; $R^3$ = 1-methyltetrazol-5-ylthiomethyl; n = 1) was treated in the same manner as Example 2 to give a compound of general formula (I) (A = cephalosporin derivative residue; $R^1$ = H; $R^2$ = thiolacetamido; $R^3$ = 1-methyltetrazol-5-ylthiomethyl; n = 1) in a yield of 89%. The NMR spectrum of this product compound supported it chemical structure.

NMR (acetone-$d_6$/methanol-$d_4$) $\delta$ ppm:
3.84 and 4.37 (ABq, 2H, J = 16 Hz)
3.92 (s, 3H)
4.01 (s, 2H)

4.30 and 4.73 (ABq, 2H, J = 14 Hz)
4.60 (d, 1H, J = 5 Hz)
5.56 (d, 1H, J = 5 Hz)
6.85-7.45 (m, 3H)

Example 17

A compound of general formula (II) (A = 1-oxacephem derivative residue; X = diphenylmethyl; $R^1$ = methoxy; $R^2$ = $\alpha$-(p-methoxybenzyloxycarbonyl)phenylacetamido;$R^3$ = 1-methyltetrazol-5-ylthiomethyl) was treated in the same manner as Example 14 to give a compound of general formula (I) (A = 1-oxacephem derivative residue; $R^1$ = methoxy; $R^2$ = $\alpha$-carboxyphenylacetamido; $R^3$ = 1-methyltetrazol-5-ylthiomethyl) in a yield of 85%. The NMR spectrum of this product compound was in agreement with that described in the literature.

Example 18

A compound of general formula (II) (A = penicillin derivative residue; X = diphenylmethyl; $R^1$ = H; $R^2$ = phenylacetamido; n = 1) was treated in the same manner as Example 2 to give a compound of general formula (I) (A = penicillin derivative residue; $R^1$ = H; $R^2$ = phenylacetamido; n = 1) in a yield of 86%. The NMR spectrum of this product compound was in agreement with that described in the literature.

Example 19

A compound of general formula (II) (A = penicillin derivative residue; X = diphenylmethyl; $R^1$ = H; $R^2$ = phenylacetamido; n = 0) was treated in the same manner as Example 2 to give a compound of general formula (I) [A = penicillin derivative residue; $R^1$ = H; $R^2$ = phenylacetamido; n = 0) in a yield of 90%. The NMR spectrum of this product compound was in agreement with that described in the literature.

Example 20

A compound of general formula (II) (A = 1-carbacephem derivative residue; X = diphenylmethyl; $R^1$ = H; $R^2$ = thiolacetamido; $R^3$ and $R^4$ each is H) was treated in the same manner as Example 2 to give a compound of general formula (I) (A = 1-carbacephem derivative residue; $R^1$ = H; $R^2$ = thiolacetamido; $R^3$ and $R^4$ each is H) in a yield of 94%. The NMR spectrum of this product compound was in agreement with that described in the literature.

Example 21

A compound of general formula (II) (A = penicillin derivative residue; X = p-methoxybenzyl; $R^1$ = H; $R^2$ = Br; n = 0) was treated in the same manner as Example 2 to give a compound of general formula (I) (A = penicillin derivative residue; $R^1$ = H; $R^2$ = Br; n = 0) in a yield of 89%. The NMR spectrum of this product compound supported its chemical structure.
NMR ($CDCl_3$) $\delta$ ppm:
1.57 (s, 3H)
1.65 (s, 3H)
4.58 (s, 1H)
4.84 (d, 1H, J = 1.5 Hz)
5.40 (d, 1H, J = 1.5 Hz)

## Claims

1. A method of producing a $\beta$-lactam derivative characterized by reacting a $\beta$-lactam derivative having a protected carboxyl group which has the general formula

A-COO-X

wherein A is a $\beta$-lactam derivative residue; X is a benzyl group having a hydroxyl group, lower alkyl groups or lower alkoxy groups as electron-donating phenyl ring substituents,

a diphenylmethyl group which may have a hydroxyl group, lower alkyl groups or lower alkoxy groups as electron-donating phenyl ring substituents, or a heteroatom within the molecule, with a phenol compound which may have halogen atoms, lower alkyl groups and lower alkoxy groups as substituents to give a β-lactam derivative of the general formula:

A-COOH

wherein A has the same meaning as above.

**2.** The method according to claim 1 wherein the β-lactam derivative residue designated by A is a cephalosporin derivative residue, an 3-exomethylenecepham derivative residue, an 1-oxacephem derivative residue, a 1-carbacephem derivative residue or a penicillin derivative residue.

**3.** The method according to claim 1 or 2 wherein said phenol compound is at least one member selected from the group consisting of phenol, o-chlorophenol, m-chlorophenol, p-chlorophenol, o-cresol, m-cresol, p-cresol and m-methoxyphenol.

**4.** The method according to any of claims 1, 2 and 3 wherein about 0.5 to 500 weight parts of said phenol compound is reacted with the starting material β-lactam derivative.

## Patentansprüche

**1.** Verfahren zur Herstellung eines β-Lactamderivates, gekennzeichnet durch das Umsetzen eines β-Lactamderivates mit einer geschützten Carboxylgruppe, das die allgemeine Formel hat:

A-COO-X

worin A der Rest eines β-Lactamderivates ist, X eine Benzylgruppe mit einer Hydroxylgruppe, Niederalkylgruppen oder Niederalkoxygruppen als elektronenschiebende Phenylringsubstituenten, eine Diphenylmethylgruppe, die eine Hydroxylgruppe, Niederalkylgruppen oder Niederalkoxygruppen als elektronenschiebende Phenylringsubstituenten aufweisen kann, oder ein Heteroatom innerhalb des Moleküls ist,
mit einer Phenolverbindung, die Halogenatome, Niederalkylgruppen oder Niederalkoxygruppen als Substituenten aufweisen kann, zur Bildung eines β-Lactamderivates der allgemeinen Formel

A-COOH ,

worin A die gleiche Bedeutung wie oben hat.

**2.** Verfahren gemäß Anspruch 1, worin der durch A bezeichnete Rest des β-Lactamderivates ein Rest eines Cephalosporinderivates, ein Rest eines 3-Exomethylencephamderivates, ein Rest eines 1-Oxacephemderivates, ein Rest eines 1-Carbacephemderivates oder ein Rest eines Penicillinderivates ist.

**3.** Verfahren gemäß Anspruch 1 oder 2, worin die Phenolverbindung mindestens eine Verbindung, ausgewählt aus der aus Phenol, o-Chlorphenol, m-Chlorphenol, p-Chlorphenol, o-Kresol, m-Kresol, p-Kresol und m-Methoxyphenol bestehenden Gruppe, ist.

**4.** Verfahren gemäß irgendeinem der Ansprüche 1, 2 und 3, worin etwa 0,5 bis 500 Gew.-Teile der Phenolverbindung mit dem β-Lactamderivat-Ausgangsmaterial umgesetzt werden.

## Revendications

**1.** Procédé de production d'un dérivé à cycle β-lactame, caractérisé par la réaction d'un dérivé à cycle β-lactame ayant un groupe carboxy protégé qui a la formule générale

A-COO-X

dans laquelle A est un résidu de dérivé à cycle β-lactame; X est un groupe benzyle ayant un groupe

hydroxy, des groupes alkyles inférieurs ou des groupes alcoxy inférieurs en tant que substituants donneurs d'électrons du cycle phényle, un groupe diphénylméthyle qui peut avoir un groupe hydroxy, des groupes alkyles inférieurs ou des groupes alcoxy inférieurs en tant que substituants donneurs d'électrons du cycle phényle, ou un hétéroatome à l'intérieur de la molécule, avec un composé du phénol qui peut avoir des atomes d'halogène, des groupes alkyles inférieurs ou des groupes alcoxy inférieurs en tant que substituants, pour donner un dérivé à cycle $\beta$-lactame de formule générale

A-COOH

dans laquelle A est tel que défini plus haut.

**2.** Procédé suivant la revendication 1, dans lequel le résidu A de dérivé à cycle $\beta$-lactame est un résidu de dérivé de céphalosporine, un résidu de dérivé de 3-exométhylène-céphame, un résidu de dérivé de 1-carbacéphème ou un résidu de dérivé de pénicilline.

**3.** Procédé suivant la revendication 1 ou la revendication 2, dans lequel ce composé du phénol est au moins un membre choisi dans le groupe consistant en phénol o-chlorophénol, m-chlorophénol, p-chlorophénol, o-crésol, m-crésol, p-crésol et m-méthoxyphénol.

**4.** Procédé suivant l'une quelconque des revendications 1, 2 ou 3, dans lequel on fait réagir d'environ 0,5 à 500 parties en poids de ce composé du phénol avec le dérivé à cycle $\beta$-lactame de départ.